(19) Europäisches Patentamt
European Patent Office
Office européen des brevets

(11) Veröffentlichungsnummer : **0 030 761**
**B1**

(12) **EUROPÄISCHE PATENTSCHRIFT**

(45) Veröffentlichungstag der Patentschrift : **21.03.84**

(21) Anmeldenummer : **80201158.5**

(22) Anmeldetag : **05.12.80**

(51) Int. Cl.³ : **C 07 C 43/184**, C 09 K 3/34, C 07 C 43/21, C 07 C 93/12, C 07 C121/46, C 07 C 41/16, C 07 C 41/00

(54) **Anisotrope Cyclohexyläther.**

(30) Priorität : **17.12.79 CH 11139/79**

(43) Veröffentlichungstag der Anmeldung : **24.06.81 Patentblatt 81/25**

(45) Bekanntmachung des Hinweises auf die Patenterteilung : **21.03.84 Patentblatt 84/12**

(84) Benannte Vertragsstaaten : **CH DE GB LI**

(56) Entgegenhaltungen :
**DE-A- 2 837 218**
**DE-B- 2 429 093**

(73) Patentinhaber : **Merck Patent Gesellschaft mit beschränkter Haftung**
**Frankfurter Strasse 250**
**D-6100 Darmstadt (DE)**

(72) Erfinder : **Osman, Maged A., Dr.**
**Lerchenrain 1**
**CH-8046 Zürich (CH)**
Erfinder : **Révész, Laszlo, Dr.**
**Lanzkronstrasse 23**
**CH-4056 Basel (CH)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 030 761 B1

## Anisotrope Cyclohexyläther

Die Erfindung betrifft neue anisotrope Verbindungen, und zwar die neue Klasse der Cyclohexyl-cyclohexylmethyläther sowie Flüssigkristall-(FK)-mischungen, die Cyclohexyl-cyclohexylmethyläther enthalten und als Dielektrikum für Flüssigkristallanzeigen verwendet werden. Die Erfindung betrifft ferner Verfahren zur Herstellung der neuen anisotropen Verbindungen.

Anisotrope Biphenylverbindungen der Formel (10)

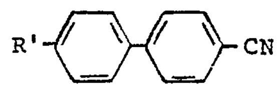

(10)

in der R' z. B. einen Alkylrest bedeutet, sind z. B. aus der DE-OS 2 356 085 bekannt und gehören zu den für FK-Mischungen am meisten verwendeten Substanzen.

Aus der DE-OS 2 636 684 ist ferner bekannt, dass sich die vergleichsweise hohen Viskositäts- und Δn-Werte der Verbindungen (10) vermindern lassen, wenn der eine Phenylenring durch einen trans-1,4-Cyclohexylenring ersetzt wird, was zu Phenylcyclohexanen der Formel (20)

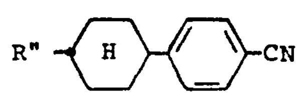

(20)

führt, in der R" die gleiche Bedeutung wie R' in Formel (10) hat.

Die Δn-Werte von anisotropen Komponenten von FK-Mischungen stellen bekanntlich ein Mass für die optische Anisotropie dar und werden als Differenz der Brechungsindizes parallel bzw. senkrecht zur Molekülachse der in Frage stehenden Substanz als $\Delta n = n_{\parallel}\text{-}n_{\perp}$ ausgedrückt.

Für manche Typen von FK-Anzeigen, insbesondere die sogenannten « Guest-host »-Effektzellen, werden nematische FK-Phasen mit möglichst kleinen Δn-Werten, z. B. nicht über 0,1, benötigt. Durch den Ersatz des einen Phenylenringes in den Verbindungen (10) durch den trans-Cyclohexylenring wird der Δn-Wert praktisch auf die Hälfte reduziert ($\Delta n \simeq 0{,}22$ bei Verbindungen der Formel (10), $\Delta n \simeq 0{,}12$ bei Verbindungen der Formel (20)).

Wird nun auch der zweite Phenylenring der anisotropen Verbindungen (10) bzw. der verbleibende Phenylenring der Verbindungen (20) durch den Cyclohexylenring ersetzt, so gelangt man zu den aus der DE-OS 2 702 598 bekannten Cyclohexylcyclohexanen der Formel (30)

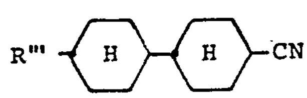

(30)

in der R"' die für R' in Formel (10) angegebene Bedeutung hat; wiederum wird hier durch der Δn-Wert vermindert, und zwar auf Werte unter 0,1, typisch etwa auf $\Delta n = 0{,}06$.

Nachteilig bei den Verbindungen (30) ist aber die Tatsache (siehe R. Pohl et al, Phys. Letter 65A (2), 169/1978), dass die Hydrierung des zweiten Phenylenringes eine ausgeprägte Tendenz zur Bildung von smektischen Phasen verursacht.

Schliesslich gehören zum Stand der Technik noch die in Mol. Cryst. Liq. Cryst. 56 (1979) 105 beschriebenen Cyclohexylcyclohexanoate der Formel (40)

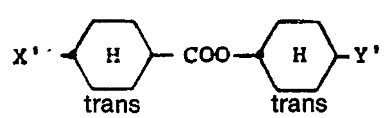

(40)

in der X' und Y' Alkyl-, Alkylcyclohexyl- oder Nitrilgruppen sind.

Die Verbindungen (40) haben vorteilhaft niedrige Δn-Werte, eine relativ geringe Viskosität und im Gegensatz zu den bekannten Verbindungen (30) keine ausgeprägte Tendenz zur Bildung smektischer Phasen, so dass die Carboxylgruppe als vergleichsweise kritischer Bestandteil für die vorteilhaften Eigenschaften der Verbindungen (40) anzusehen war.

Die Carboxylgruppe als Brücke zwischen den Cyclohexylringen (A) und (B) der Formel (40) ist jedoch verhältnismässig reaktiv bzw. hydrolyseempfindlich.

Aufgabe der vorliegenden Erfindung sind neue anisotrope Verbindungen, welche die Vorteile der Cyclohexylcyclohexanoate der Formel (40) bieten, aber im Vergleich zu diesen eine höhere chemische Stabilität besitzen.

Zur Aufgabe der Erfindung gehören ferner FK-Mischungen, welche neue anisotrope Verbindungen enthalten, und Verfahren zur Herstellung neuer anisotroper Verbindungen.

Es wurde gefunden, dass die Aufgaben der Erfindung dadurch erreicht werden, dass man die Carboxylgruppe, welche die Cyclohexylringe (A) und (B) der Verbindungen (40) miteinander verbindet, durch eine Aetherbrücke der Formel —$CH_2O$— ersetzt. Ueberraschenderweise, d. h. im Gegensatz zur fachmännischen Erwartung, haben die Aether allgemein niedrigere Schmelzpunkte als die entsprechenden Carboxylverbindungen (40).

Die neuen anisotropen Verbindungen gemäss der vorliegenden Erfindung haben die Formel (1)

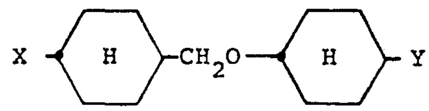

trans trans

(1)

in der X und Y jeweils Wasserstoffatome, Alkylgruppen mit 1 bis 12 C-Atomen, Alkoxygruppen mit 1 bis 12 C-Atomen, Monoalkylaminogruppen mit 1 bis 12 C-Atomen, Nitrilgruppen, Nitrogruppen oder Halogenatome oder cyclische Reste der Formel (1a) oder (1b)

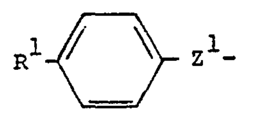

(1a)

oder

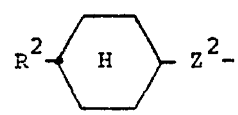

trans (1b)

bedeuten, in der $R^1$ und $R^2$ Wasserstoffatome, Alkylgruppen mit 1 bis 12 C-Atomen, Alkoxygruppen mit 1 bis 12 C-Atomen, Monoalkylaminogruppen mit 1 bis 12 C-Atomen, Nitrilgruppen, Nitrogruppen oder Halogenatome bedeuten und $Z^1$ und $Z^2$ einfache Kovalenzbindungen oder Gruppen der Formeln —COO—, —OOC—, —$CH_2O$— oder —$OCH_2$— darstellen, mit der Massgabe, dass nur eine der Gruppen X, Y einen cyclischen Rest der Formeln (1a) oder (1b) bedeutet.

Der Alkylteil in den Alkyl-, Alkoxy- oder N-Monoalkylaminoresten von X, Y, $R^1$ bzw. $R^2$ ist vorzugsweise geradkettig ; häufig werden hierfür Alkylteile mit 3 bis 10 C-Atomen, insbesondere 3 bis 8 C-Atomen, bevorzugt. Als Halogen für X, Y, $R^1$ bzw. $R^2$ werden meist Cl und Br bevorzugt.

Ferner werden häufig solche Verbindungen (1) bevorzugt, bei welchen X ungleich Y ist.

Mit den neuen anisotropen Verbindungen können FK-Mischungen hergestellt werden, die überwiegend bzw. praktisch vollständig (z. B. bis 90 Gew.%) aus Verbindungen (1) in Mischung miteinander bestehen oder mindestens eine Verbindung (1) als Komponente, z. B. in Anteilen von 2 bis 40 Gew.%, bezogen auf die FK-Mischung, enthalten, z. B. mit bekannten anisotropen Verbindungen oder/und anderen für FK-Mischungen geeigneten Komponenten.

Die neuen anisotropen Verbindungen (1) bzw. die damit hergestellten FK-Mischungen eignen sich allgemein für nematische Phasen mit kleiner optischer Anisotropie und niedriger Viskosität ; wenn die erfindungsgemässe FK-Mischung bzw. die erfindungsgemässen Verbindungen (1) eine positive DK-Anisotropie besitzen, eignen sich die nematischen Phasen besonders für die bekannten FK-Anzeigen in Form von Drehzellen im Multiplexbetrieb sowie für die bekannten FK-Anzeigen, die aufgrund des sogenannten Guest-Host-Effektes arbeiten. Wenn die erfindungsgemässe FK-Mischung bzw. die erfindungsgemässen Verbindungen (1) eine negative DK-Anisotropie besitzen, eignen sich die damit gebildeten nematischen Phasen besonders für bekannte FK-Anzeigen, die nach dem inversen Guest-Host-Effekt, als dynamische Streuzellen (DS) oder als sogenannte DDM-Zellen arbeiten.

Die neuen Verbindungen (1) können dadurch erhalten werden, dass man die entsprechenden, z. B. gemäss der oben angegebenen Literaturstelle erhältlichen Cyclohexylcyclohexanoate reduziert, d. h.

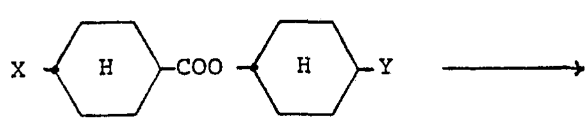

(1)

worin X und Y die oben angegebene Bedeutung haben, z. B. mit Hilfe von $BF_3/NaBH_4$ nach der an sich bekannten Methode (siehe z. B. G. R. Pettit und D. M. Piatate, J. Org. Chem. 27 (1962) 2127) zur reduktiven Umwandlung von Ester in Aether.

Ferner können die neuen Verbindungen (1) auch durch Kondensation bzw. Verätherung einer entsprechenden p-X-trans-Cyclohexylmethylverbindung der Formel (3a) mit einem entsprechenden p-Y-trans-Cyclohexyloxy-Verbindungen der Formel (3b) erhalten werden, d. h.

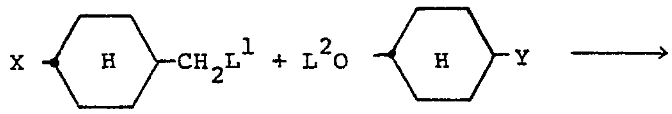 (1)

trans (3a) trans (3b)

worin X und Y die oben angegebene Bedeutung haben und $L^1$, $L^2$ Abgangsgruppen bzw. Abgangsatome sind, die miteinander bei der Kondensation $L^1L^2$ bilden. Wenn $L^1$ z. B. Halogen, wie Br, ist kann die Kondensation mit $L^2 = H$ unter Bildung von Halogenwasserstoff als Nebenprodukt $L^1L^2$ erfolgen, der in üblicher Weise mit organischer Substanz, z. B. Pyridin, oder anorganischer Substanz, z. B. Soda, zur Förderung der Reaktion/gebunden werden kann.

Eine zur Herstellung der neuen Verbindungen (1) durch Kondensation bzw. Verätherung geeignete Methode ist von G. W. Gray und D. G. McDonnell in Mol. Cryst. Liqu. Cryst. 53 (1979) 147-166 für Cyclohexylmethyläther mit aromatischem (Phenylen, Biphenylen oder Naphthylen) Ring an der Aethergruppe, d. h. für Verbindungen der Formel (50)

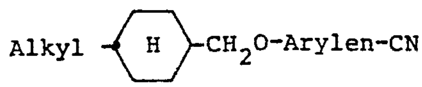 (50)

trans

die aber allgemein höhere Schmelzpunkte haben, als die entsprechenden Carboxylverbindungen der Formel (60)

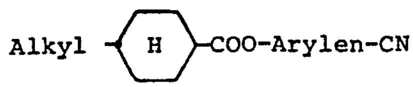 (60)

trans

Ueberraschenderweise haben die erfindungsgemässen Aether der Formel (1) aber allgemein tiefere Schmelzpunkte, als die entsprechenden Cyclohexylcyclohexanoate der Formel (40).

Die Ausgangsverbindung (3a) bzw. (3b) für die Synthese der erfindungsgemässen Verbindungen (1) durch Kondensation bzw. Verätherung ist bekannt oder kann analog wie die bekannten Verbindungen hergestellt werden.

Die Herstellung erfindungsgemässer Verbindungen (1) ist in den folgenden Beispielen erläutert.

Beispiel 1

Herstellung von trans-4-Phenylcyclohexyl-trans-4'-n-propylcyclohexylmethyläther der Formel (11)

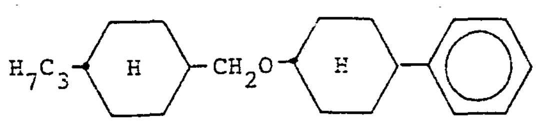 (11)

trans trans

Unter Eiskühlung wurden 0,8 g (0,021 Mol) $NaBH_4$ in 70 ml Diethylenglycoldimethyläther vorgelegt und gleichzeitig mit 4,5 g (0,0137 Mol) trans-4-Phenylcyclohexyl-trans-4'-n-propylcyclohexanoat in 75 ml Tetrahydrofuran bzw. 37 ml (0,29 Mol) Bortrifluorätherat in 75 ml Tetrahydrofuran versetzt, wobei die Temperatur nicht über 5 °C stieg. Nach beendeter Zugabe wurde das Reaktionsgemisch auf Raumtemperatur gebracht und während 1 Std. auf Rückflusstemperatur erwärmt. Zur Aufarbeitung wurden 100 ml 2 n HCl zugegeben und die organischen Lösungsmittel abdestilliert. Die wässrige Phase wurde dreimal mit Methylenchlorid extrahiert, die organischen Phasen mit Kochsalzlösung gewaschen, über $Na_2SO_4$ getrocknet und eingeengt. Das kristalline Rohprodukt wurde über Kieselgel chromatographiert und aus Ethanol umkristallisiert. $T_k$ 50,5 °C, $T_c$ (22 °C).

Der $T_k$-Wert der entsprechenden Cyclohexylcyclohexanoatverbindung (40) beträgt dagegen 84,4 °C, der $T_c$-Wert (69,5 °C). Wie üblich bedeutet hierbei $T_k$ die Temperatur des Ueberganges aus der kristallinen in die flüssige Phase und $T_c$ den Klärpunkt. Ein in Klammern angegebener $T_c$-Wert bedeutet Messung im unterkühlten Zustand.

Beispiel 2

Herstellung von trans-(4''-n-Pentyl)-4'-phenylcyclohexylmethyl-trans-4-n-propylcyclohexyläther der Formel (12)

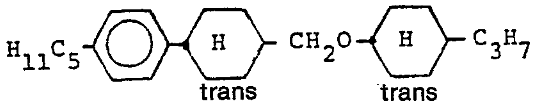
trans trans

(12)

0,8 g (0,021 Mol) $NaBH_4$ wurden unter Eiskühlung in 70 ml Diethylenglycoldimethyläther vorgelegt und gleichzeitig mit 5,5 g (0,0137 Mol) trans-4-n-Propylcyclohexyl-trans-(4''-n-pentyl)-4'-phenylcyclohexanoat in 75 ml Tetrahydrofuran bzw. 37 ml (0,29 Mol) Bortrifluoritherat in 75 ml Tetranhydrofuran versetzt, wobei die Temperatur nicht über 5 °C stieg. Nach beendeter Zugabe wurde das Reaktionsgemisch auf Raumtemperatur gebracht und während 1 Std. unter Rückflusstemperatur erwärmt. Zur Aufarbeitung wurden 100 ml 2 n HCl zugegeben und die organischen Lösungsmittel abdestilliert. Die wässrige Phase wurde dreimal mit Methylenchlorid extrahiert, die organischen Phasen mit Kochsalzlösung gewaschen, über $Na_2SO_4$ getrocknet und eingeengt. Zur Reinigung wurde das Produkt, die Verbindung (12), über Kieselgel chromatographiert und aus Ethanol umkristallisiert. $T_c$ 87,7 °C.

Die Verbindung (12) zeigte eine ausgeprägte Tendenz zur Bildung unterkühlter Schmelzen und ist bis 0 °C nicht gefroren. Hingegen zeigt das entsprechende Cyclohexylcyclohexanoat einen $T_K$-Wert von 84,2 °C und einen $T_c$-Wert von 177,3 °C.

Beispiel 3

Die gemäss Beispiel 1 durch Reduktion des entsprechenden Cyclohexylcyclohexanoates hergestellte erfindungsgemässe Verbindung der Formel (11) wurde auch durch Kondensation bzw. Verätherung entsprechender Verbindungen der Formeln (3a) und (3b) hergestellt. Hierzu wurde wie folgt gearbeitet :

(A) Herstellung von trans-4-n-Propylcyclohexylmethanol der Formel (31)

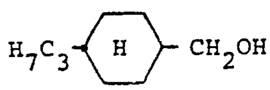
trans

(31)

23,8 g (0,14 Mol) trans-4-n-Propylcyclohexancarbonsäure wurden in 150 ml trockenem Aether gelöst und zu einer Suspension von 11,4 g (0,3 Mol) $LiAlH_4$ in 300 ml Aether unter Rühren zugetropft. Nach der Zugabe wurde während 2 Std. auf Rückfluss erwärmt und danach unter Kühlen das überschüssige $LiAlH_4$ mit Wasser zersetzt. Das Reaktionsgemisch wurde dann auf 20 %ige Salzsäure gegossen und gerührt, bis sich die anorganischen Salze lösten. Das Produkt der Formel (31) wurde mit Diethyläther extrahiert, die organischen Phasen mit Wasser gewaschen, über $MgSO_4$ getrocknet und eingeengt.

(B) Herstellung von trans-4-n-Propylcyclohexylmethylbromid der Formel (32)

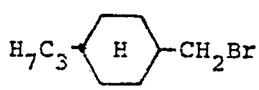
trans

(32)

15,6 g (0,1 Mol) der gemäss Abschnitt (A) erhaltenen Verbindung (31) wurden bei 0 °C in 150 ml Pyridin-Benzol-Mischung (1 : 1) vorgelegt und unter Rühren tropfenweise mit 9,0 g (0,033 Mol) $PBr_3$ versetzt. Das Reaktionsgemisch wurde 2 Std. bei 0 °C, dann 2 Std. bei Raumtemperatur gerührt, auf Eis/2 n HCl gegossen und mit Diethyläther extrahiert. Die Aetherextrakte wurden je einmal mit 2 n HCl, 2 n NaOH und Kochsalzlösung gewaschen, über $Na_2SO_4$ getrocknet und eingeengt. Zur Reinigung wurde das Bromid der Formel (32) über Kieselgel chromatographiert.

(C) Herstellung der Verbindung (11) durch Verätherung

1,56 g (0,065 Mol) NaH wurden in 30 ml trockenem Tetrahydrofuran (THF) vorgelegt, mit 11,4 g (0,065 Mol) trans-4-Phenylcyclohexanol in 20 ml THF langsam unter Kontrolle der Wasserstoffentwicklung versetzt und bei Raumtemperatur über Nacht gerührt. 14,2 g (0,065 Mol) des gemäss Abschnitt (B) erhaltenen Bromids der Formel (32) in 20 ml THF wurden langsam zugetropft und das Ganze 2 Std. auf Rückflusstemperatur erwärmt. Zur Aufarbeitung wurde das THF abdestilliert, der Rückstand auf Eiswasser gegossen und mit Diethyläther extrahiert, die Aetherphasen mit Kochsalzlösung gewaschen, über $Na_2SO_4$ getrocknet und eingeengt. Das Rohprodukt der Formel (11) wurde über Kieselgel gereinigt und aus Ethanol umkristallisiert. $T_k$ 50,5 °C, $T_c$ (22 °C).

Beispiel 4

Die gemäss Beispiel 2 durch Reduktion des entsprechenden Cyclohexylcyclohexanoates hergestellte

Verbindung der Formel (12) wurde auch durch Kondensation bzw. Verätherung entsprechender Verbindungen der Formeln (3a) und (3b) hergestellt. Hierzu wurde wie folgt gearbeitet :

(A) Herstellung von trans-(4'-n-Pentyl)-4-phenylcyclohexylmethanol der Formel (33)

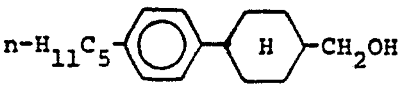
trans

(33)

38,3 g (0,14 Mol) trans-(4'-n-Pentyl)-4-phenylcyclohexancarbonsäure wurden in 150 ml trockenem Diethyläther gelöst und zu einer Suspension von 11,4 g (0,3 Mol) $LiAlH_4$ in 300 ml Aether unter Rühren zugetropft. Nach beendeter Zugabe wurde während 2 Std. auf Rückflusstemperatur erwärmt und danach unter Kühlen das überschüssige $LiAlH_4$ mit Wasser zersetzt. Das Reaktionsgemisch wurde darauf auf 20 %ige Salzsäure gegossen und gerührt, bis sich die anorganischen Salze lösten. Das Produkt wurde mit Diethyläther extrahiert, die Aetherphasen mit Wasser gewaschen, über $MgSO_4$ getrocknet und eingeengt.

(B) Herstellung von trans-(4'-n-Pentyl)-4-phenylcyclohexylmethylbromid der Formel (34)

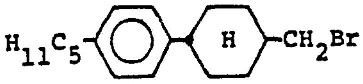
trans

(34)

26,0 g (0,1 Mol) der gemäss Abschnitt (A) erhaltenen Verbindung (33) wurden bei 0 °C in 150 ml Pyridin-Benzol-Mischung (1 : 1) vorgelegt und unter Rühren tropfenweise mit 9,0 g (0,033 Mol) $PBr_3$ versetzt. Das Reaktionsgemisch wurde 2 Std. bei 0 °C, dann 2 Std. bei Rückflusstemperatur gerührt, auf Eis/2 n HCl gegossen und mit Diethyläther extrahiert. Die Aetherextrakte wurden je einmal mit 2 n HCl, 2 n NaOH und Kochsalzlösung gewaschen, über $Na_2SO_4$ getrocknet und eingeengt. Zur Reinigung wurde das Bromid (34) über Kieselgel chromatographiert und aus Ethanol umkristallisiert.

(C) Herstellung der Verbindung (12) durch Verätherung

1,56 g (0,065 Mol) NaH wurden in 30 ml trockenem THF vorgelegt, mit 9,23 g (0,065 Mol) trans-4-n-Propylcyclohexanol in 20 ml THF langsam unter Kontrolle der Wasserstoffentwicklung versetzt und bei Raumtemperatur über Nacht gerührt. 21 g (0,065 Mol) der gemäss Abschnitt (B) erhaltenen Verbindung (34) in 20 ml THF wurden langsam zugetropft und das Ganze 2 Std. auf Rückflusstemperatur erhitzt. Zur Aufarbeitung wurde das THF abdestilliert, der Rückstand auf Eiswasser gegossen und mit Diethyläther extrahiert, die Aetherphasen mit Kochsalzlösung gewaschen, über $Na_2SO_4$ getrocknet und eingeengt. Das Rohprodukt wurde über Kieselgel gereinigt und aus Ethanol umkristallisiert. Die physikalischen Daten der erhaltenen Verbindung (12) sind in Beispiel 2 angegeben.

Beispiele 5-73

In analoger Weise wie in den Beispielen 1-4 beschrieben sind auch die folgenden erfindungsgemässen anisotropen Verbindungen der Formel (1) erhältlich :

Beispiele

5 trans-4-Phenylcyclohexyl-trans-4'-n-propylcyclohexylmethyläther
6 trans-4-Phenylcyclohexyl-trans-4'-n-pentylcyclohexylmethyläther
7 trans-4-Phenylcyclohexyl-trans-4'-n-heptylcyclohexylmethyläther
8 trans-4-Phenylcyclohexyl-trans-4'-n-nonylcyclohexylmethyläther
9 trans-(4"-n-Propyl)-4'-phenylcyclohexylmethyl-trans-4-n-propylcyclohexyläther
10 trans-(4"-n-Pentyl)-4'-phenylcyclohexylmethyl-trans-4-n-propylcyclohexyläther
11 trans-(4"-n-Heptyl)-4'-phenylcyclohexylmethyl-trans-4-n-propylcyclohexyläther
12 trans-(4"-n-Nonyl)-4'-phenylcyclohexylmethyl-trans-4-n-propylcyclohexyläther
13 trans-(4"-n-Propyl)-4'-phenylcyclohexylmethyl-trans-4-n-pentylcyclohexyläther
14 trans-(4"-n-Propyl)-4'-phenylcyclohexylmethyl-trans-4-n-heptylcyclohexyläther
15 trans-(4"-n-Propyl)-4'-phenylcyclohexylmethyl-trans-4-n-nonylcyclohexyläther
16 trans-(4"-n-Pentyl)-4'-phenylcyclohexylmethyl-trans-4-n-pentylcyclohexyläther
17 trans-(4"-n-Pentyl)-4'-phenylcyclohexylmethyl-trans-4-n-heptylcyclohexyläther

18 trans-(4"-n-Pentyl)-4'-phenylcyclohexylmethyl-trans-4-n-nonylcyclohexyläther
19 trans-(4"-n-Heptyl)-4'-phenylcyclohexylmethyl-trans-4-n-pentylcyclohexyläther
20 trans-(4"-n-Heptyl)-4'-phenylcyclohexylmethyl-trans-4-n-heptylcyclohexyläther
21 trans-(4"-n-Heptyl)-4'-phenylcyclohexylmethyl-trans-4-n-nonylcyclohexyläther
22 trans-(4"-n-Nonyl)-4'-phenylcyclohexylmethyl-trans-4-n-pentylcyclohexyläther
23 trans-(4"-n-Nonyl)-4'-phenylcyclohexylmethyl-trans-4-n-heptylcyclohexyläther
24 trans-(4"-n-Nonyl)-4'-phenylcyclohexylmethyl-trans-4-nonylcyclohexyläther
25 trans-4'-Methylcyclohexylmethyl-trans-4-n-propylcyclohexyläther
26 trans-4'-n-Propylcyclohexylmethyl-trans-4-n-propylcyclohexyläther
27 trans-4'-n-Pentylcyclohexylmethyl-trans-4-n-propylcyclohexyläther
28 trans-4'-n-Heptylcyclohexylmethyl-trans-4-n-propylcyclohexyläther
29 trans-4'-n-Nonylcyclohexylmethyl-trans-4-n-propylcyclohexyläther
30 trans-4'-n-Propylcyclohexylmethyl-trans-4-methylcyclohexyläther
31 trans-4'-n-Propylcyclohexylmethyl-trans-4-n-pentylcyclohexyläther
32 trans-4'-n-Propylcyclohexylmethyl-trans-4-n-heptylcyclohexyläther
33 trans-4'-n-Propylcyclohexylmethyl-trans-4-n-nonylcyclohexyläther
34 trans-4'-Methylcyclohexylmethyl-trans-4-n-pentylcyclohexyläther
35 trans-4'-n-Pentylcyclohexylmethyl-trans-4-n-pentylcyclohexyläther
36 trans-4'-n-Heptylcyclohexylmethyl-trans-4-n-pentylcyclohexyläther
37 trans-4'-n-Nonylcyclohexylmethyl-trans-4-n-pentylcyclohexyläther
38 trans-4'-n-Pentylcyclohexylmethyl-trans-4-methylcyclohexyläther
39 trans-4'-n-Pentylcyclohexylmethyl-trans-4-n-heptylcyclohexyläther
40 trans-4'-n-Pentylcyclohexylmethyl-trans-4-n-nonylcyclohexyläther
41 trans-4'-Methylcyclohexylmethyl-trans-4-n-heptylcyclohexyläther
42 trans-4'-n-Heptylcyclohexylmethyl-trans-4-n-heptylcyclohexyläther
43 trans-4'-n-Nonylcyclohexylmethyl-trans-4-n-heptylcyclohexyläther
44 trans-4'-n-Heptylcyclohexylmethyl-trans-4-methylcyclohexyläther
45 trans-4'-n-Heptylcyclohexylmethyl-trans-4-n-nonylcyclohexyläther
46 trans-4'-Methylcyclohexylmethyl-trans-4-n-nonylcyclohexyläther
47 trans-4'-n-Nonylcyclohexylmethyl-trans-4-n-nonylcyclohexyläther
48 trans-4'-n-Nonylcyclohexylmethyl-trans-4-methylcyclohexyläther
49 trans-4'-Phenylcyclohexyl-trans-4-ethoxycyclohexylmethyläther
50 trans-4'-Phenylcyclohexyl-trans-4-n-butyloxycyclohexylmethyläther
51 trans-4'-Phenylcyclohexyl-trans-4-n-hexyloxycyclohexylmethyläther
52 trans-4'-Phenylcyclohexyl-trans-4-n-octyloxycyclohexylmethyläther
53 trans-(4"-Ethoxy)-4'-phenylcyclohexylmethyl-trans-4-n-propylcyclohexyläther
54 trans-(4"-n-Butyloxy)-4'-phenylcyclohexylmethyl-trans-4-n-propylcyclohexyläther
55 trans-(4"-n-Hexyloxy)-4'-phenylcyclohexylmethyl-trans-4-n-propylcyclohexyläther
56 trans-(4"-n-Octyloxy)-4'-phenylcyclohexylmethyl-trans-4-n-propylcyclohexyläther
57 trans-(4"-Ethoxy)-4'-phenylcyclohexylmethyl-trans-4-n-pentylcyclohexyläther
58 trans-(4"-n-Butyloxy)-4'-phenylcyclohexylmethyl-trans-4-n-pentylcyclohexyläther
59 trans-(4"-n-Hexyloxy)-4'-phenylcyclohexylmethyl-trans-4-n-pentylcyclohexyläther
60 trans-(4"-n-Octyloxy)-4'-phenylcyclohexylmethyl-trans-4-n-pentylcyclohexyläther
61 trans-4'-n-Propylcyclohexylmethyl-trans-4-n-pentylaminocyclohexyläther
62 trans-4'-n-Propylaminocyclohexylmethyl-trans-4-n-pentylcyclohexyläther
63 trans-4'-n-Heptylaminocyclohexylmethyl-trans-4-n-propylcyclohexyläther
64 trans-4'-Cyanocyclohexylmethyl-trans-4-n-heptylcyclohexyläther
65 trans-4'-n-Heptylcyclohexylmethyl-trans-4-cyanocyclohexyläther
66 trans-4'-n-Pentyl-4-bicyclohexyl-trans-4"-n-propylcyclohexylmethyläther
67 trans-4'-n-Propyl-4-bicyclohexyl-trans-4"-n-pentylcyclohexylmethyläther
68 trans-4'-Cyano-4-bicyclohexyl-trans-4"-n-heptylcyclohexylmethyläther
69 trans-4'-n-Pentyl-4-bicyclohexyl-trans-4"-cyanocyclohexylmethyläther
70 trans-4-n-Propylcyclohexylmethyl-trans-4'-[(trans-4"-n-pentyl)-cyclohexyloxymethyl]-cyclohexyläther
71 trans-4-n-Pentylcyclohexylmethyl-trans-4'-[(trans-4"-n-propyl)-cyclohexyloxymethyl]-cyclohexyläther
72 trans-4-Cyanocyclohexylmethyl-trans-4'-[(trans-4"-n-heptyl)-cyclohexyloxymethyl]-cyclohexyläther
73 trans-4-n-Heptylcyclohexylmethyl-trans-4'-[(trans-4"-cyano)-cyclohexyloxymethyl]-cyclohexyläther.

Beispiel 74

Herstellung von trans-4-n-Propylcyclohexylmethyl-trans-4"-propylphenyl-4'-trans-cyclohexyläther (Formel 35)

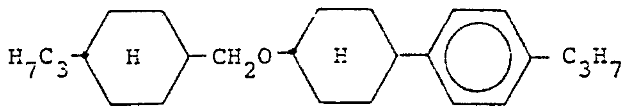 (35)

trans trans

Diese Verbindung wurde nach der in Beispiel 1 beschriebenen Methode durch Reduktion von trans-(4"-n-Propyl)-4'-phenylcyclohexyl-trans-4-n-propylcyclohexanoat hergestellt, deren Anisotropie aus den Werten $T_K = 99{,}0\ °C$, $T_s = 100{,}5\ °C$, $T_c = 102{,}0\ °C$ ersichtlich ist. Der Wert $T_s$ bedeutet das Auftreten einer smektischen Phase entsprechend K 99.0 S 100.5 N 102.0 I.

Die der Verbindung (35) analoge, d. h. um die Phenylengruppe verkürzte Verbindung von Beispiel 26 zeigt einen analogen, aber um fast 100 °C tiefer liegenden Anisotropiebereich entsprechend K 6.9 S 8.0 N 17.5 I.

Es versteht sich, dass die neuen anisotropen Verbindungen (1), unter anderem wegen ihrer geringen optischen Anisotropie und ihrer vergleichsweise niedrigen Viskosität, ein ausserordentlich breites Anwendungsspektrum haben ; je nach Wahl der Substituenten X, Y in Formel (1) im Sinne einer stärkeren oder schwächeren Polarisierung des Moleküls können die neuen anisotropen Verbindungen (1) unterschiedliche Funktionen in FK-Mischungen für FK-Anzeigen der oben genannten Arten erfüllen ; dementsprechend lassen sich mit den anisotropen Verbindungen (1) — die einzeln oder in Mischung verwendet werden können — ganz unterschiedliche FK-Mischungen bilden, und zwar entweder zusammen mit bekannten Komponenten von FK-Mischungen oder praktisch nur aus den Verbindungen (1).

Herstellung und Verwendung von FK-Mischungen für Anzeigezellen der oben genannten Art gehören im übrigen ebenso wie der Aufbau und der Betrieb solcher Zellen zum fachmännischen Wissen und erfordern keine eingehendere Beschreibung.

**Ansprüche**

1. Anisotrope Verbindungen der Formel (1)

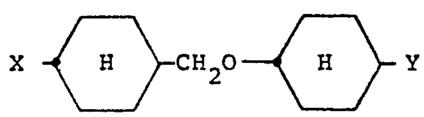 (1)

trans trans

in der X und Y jeweils Wasserstoffatome, Alkylgruppen mit 1 bis 12 C-Atomen, Alkoxygruppen mit 1 bis 12 C-Atomen, Monoalkylaminogruppen mit 1 bis 12 C-Atomen, Nitrilgruppen, Nitrogruppen oder Halogenatome oder cyclische Reste der Formel (1a) oder (1b)

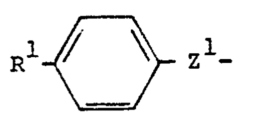 oder 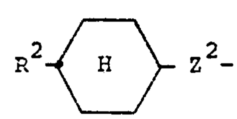

(1a) trans (1b)

bedeuten, in der $R^1$ und $R^2$ Wasserstoffatome, Alkylgruppen mit 1 bis 12 C-Atomen, Alkoxygruppen mit 1 bis 12 C-Atomen, Monoalkylaminogruppen mit 1 bis 12 C-Atomen, Nitrilgruppen, Nitrogruppen oder Halogenatome bedeuten und $Z^1$ und $Z^2$ einfache Kovalenzbindungen oder Gruppen der Formeln —COO—, —OOC—, —$CH_2O$— oder —$OCH_2$— darstellen, mit der Massgabe, dass nur eine der Gruppen X, Y einen cyclischen Rest der Formeln (1a) oder (1b) bedeutet.

2. Flüssigkristallmischung, dadurch gekennzeichnet, dass sie mindestens eine anisotrope Verbindung der Formel (1) gemäss Anspruch 1 enthält, wobei die Symbole die oben zu Formel (1) angegebenen Bedeutungen haben.

3. Verfahren zur Herstellung von anisotropen Verbindungen der Formel (1) gemäss Anspruch 1, dadurch gekennzeichnet, dass eine entsprechende Cyclohexylcyclohexanoatverbindung der Formel (3)

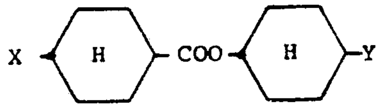 (3)

trans trans

in der X und Y die oben zu Formel (1) angegebenen Bedeutungen haben, reduziert oder die entsprechende Cyclohexylmethylverbindung der Formel (3a)

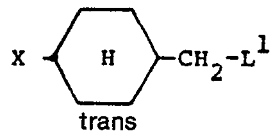
trans (3a)

in der X die oben zu Formel (1) angegebene Bedeutung hat und in der $L^1$ eine Abgangsgruppe ist, mit der entsprechenden Cyclohexylverbindung der Formel (3b)

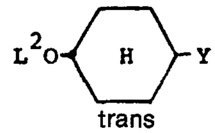
trans (3b)

in der Y die oben zu Formel (1) angegebene Bedeutung hat und in der $L^2$ eine Abgangsgruppe ist, unter Abspaltung von $L^1$-$L^2$ kondensiert wird.

**Claims**

1. Anisotropic compounds of formula (1)

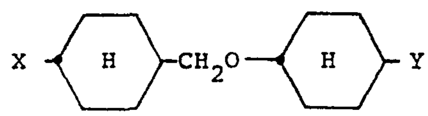
trans trans (1)

in which X and Y independently represent hydrogen, alkyl group with 1 to 12 C-atoms, alkoxy group with 1 to 12 C-atoms, monoalkylamino group with 1 to 12 C-atoms, nitrile group, nitro group, halogen, or cyclic radical of formula (1a) or (1b)

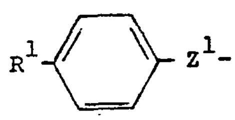
(1a)

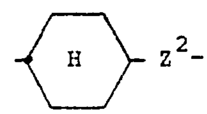
trans(1b)

in which $R^1$ and $R^2$ represent hydrogen, alkyl group with 1 to 12 C-atoms, alkoxy group with 1 to 12 C-atoms, monoalkylamino group with 1 to 12 C-atoms, nitrile group, nitro group, or halogen, and $Z^1$ and $Z^2$ represent simple covalent bonds or groups of the formulas —COO—, —OOC—, —$CH_2$O— or —O$CH_2$—, with the requirement that only one of the groups X, Y can be a cyclic radical of formula (1a) or (1b).

2. A liquid crystal mixture, containing at least one anisotropic compound of formula (1) according to Claim 1, wherein the symbols have the meanings given above for formula (1).

3. A method of producing anisotropic compounds of formula (1) according to Claim 1, comprising the step of reducing a corresponding cyclohexyl cyclohexanoate of formula (3)

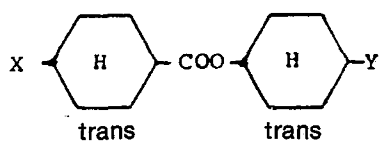
trans trans (3)

wherein X and Y have the meanings given above for formula (1), or the step of condensing the corresponding cyclohexylmethyl compound of formula (3a)

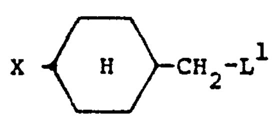
trans (3a)

wherein X has the meaning given above for formula (1) and $L^1$ is a leaving group, with the corresponding cyclohexyl compound of formula (3b)

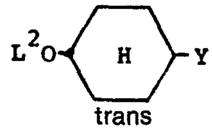

(3b)

wherein Y has the meaning given above for formula (1) and $L^2$ is a leaving group, by splitting off $L^1$-$L^2$.

**Revendications**

1. Composés anisotropes de la formule (1)

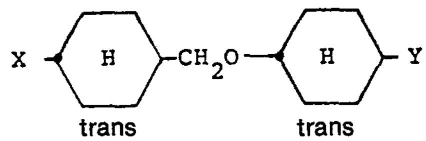

(1)

dans laquelle X et Y représentent des atomes d'hydrogène, des groupements alcoyles avec 1 à 12 atomes de C, des groupements alkoxy avec 1 à 12 atomes de C, des groupements monoalkylaminés avec 1 à 12 atomes de C, des groupements nitriles, des groupements nitro ou des atomes d'halogènes ou des radicaux cycliques de la formule (1a) ou (1b).

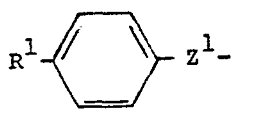

ou

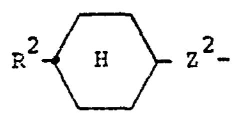

(1a) trans (1b)

dans laquelle $R^1$ et $R^2$ représentent des atomes d'hydrogène, des groupements alcoyles avec 1 à 12 atomes de C, des groupements alkoxy avec 1 à 12 atomes de C, des groupements monoalkylaminés avec 1 à 12 atomes de C, des groupements nitriles, des groupements nitro ou des atomes d'halogènes et $Z^1$ et $Z^2$ de simples liaisons covalentes ou des groupements de formules —COO—, —OOC—, —$CH_2O$— ou —$OCH_2$—, étant entendu que seul un des groupements X, Y représente un radical cyclique des formules (1a) ou (1b).

2. Mélange de cristaux liquides, caractérisé par le fait qu'il contient au minimum un composé anisotrope de la formule (1) selon la revendication 1, les symboles ayant les significations indiquées ci-dessus pour la formule (1).

3. Procédé de fabrication de composés anisotropes de la formule (1) selon la revendication 1, caractérisé par le fait qu'est réduit un composé cyclohexylcyclohexanoate correspondant de la formule (3).

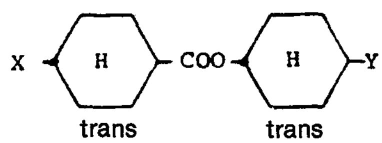

(3)

dans laquelle X et Y possèdent la signification indiquée ci-dessus pour la formule (1), ou que le composé cyclohexylméthyle correspondant de la formule (3a)

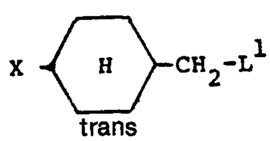

(3a)

dans laquelle X et Y possèdent la signification indiquée ci-dessus pour la formule (1), et $L^1$ est un groupement éliminable, est condensé avec le composé cyclohexyle correspondant de la formule (3b)

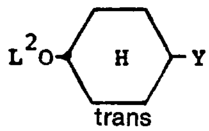

(3b)

dans laquelle Y possède la signification indiquée ci-dessus pour la formule (1) et $L^2$ est un groupement éliminable, $L^1$-$L^2$ étant éliminés.